(19) Europäisches Patentamt
European Patent Office
Office européen des brevets

(11) **EP 2 648 133 A1**

(12) **DEMANDE DE BREVET EUROPEEN**

(43) Date de publication:
**09.10.2013 Bulletin 2013/41**

(51) Int Cl.:
***G06K 9/62*** *(2006.01)*

(21) Numéro de dépôt: **12305402.5**

(22) Date de dépôt: **04.04.2012**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**

(71) Demandeur: **BIOMERIEUX
69280 Marcy-L'Etoile (FR)**

(72) Inventeurs:
• **Vervier, Kevin
38460 Annoisin-Chatelans (FR)**

• **Mahe, Pierre
38250 Lans en vercors (FR)**
• **Veyrieras, Jean-Baptiste
69001 Lyon (FR)**

(74) Mandataire: **Vuillermoz, Bruno et al
Cabinet Laurent & Charras
"Le Contemporain"
50, Chemin de la Bruyère
69574 Dardilly Cédex (FR)**

(54) **Identification de microorganismes par spectrometrie et classification structurée**

(57) La présente invention concerne un procédé d'identification par spectrométrie de microorganismes inconnus parmi un ensemble d'espèces de référence, comportant une première étape d'apprentissage supervisé d'un modèle de classification des espèces de référence, une seconde étape de prédiction d'un microorganisme inconnu à identifier comportant l'acquisition d'un spectre du microorganisme inconnu ; et l'application d'un modèle de prédiction en fonction dudit spectre et du modèle de classification afin d'inférer au moins un type de micro-organisme auquel le microorganisme inconnu appartient.

Le modèle de classification est calculé au moyen d'un algorithme SVM multi-classe structuré appliquée aux noeuds d'une représentation hiérarchique sous forme d'arbre des espèces de référence en termes d'évolution et/ou de phénotype clinique et comportant des contraintes des contraintes de marges comprenant des fonctions dites « de perte » quantifiant une proximité entre les noeuds de l'arbre.

EP 2 648 133 A1

**Description**

**DOMAINE DE L'INVENTION**

L'invention a trait au domaine de la classification de microorganismes, notamment de bactéries, au moyen de la spectrométrie.

L'invention trouve particulièrement application dans l'identification de microorganismes au moyen de la spectrométrie de masse, par exemple de type MALDI-TOF (acronyme de « *Matrix-assisted laser desorption/ionization time of flight* »)*,* de la spectrométrie vibrationnelle, et la spectroscopie par auto-fluorescence.

**ETAT DE LA TECHNIQUE**

Il est connu d'utiliser la spectrométrie ou la spectroscopie pour identifier des micro-organismes, et plus particulièrement des bactéries. Pour ce faire, un échantillon d'un micro-organisme inconnu à identifier est préparé puis un spectre de masse, vibrationnel ou de fluorescence de l'échantillon est acquis et pré-traité, notamment pour éliminer la ligne de base (communément appelé « baseline ») et pour éliminer le bruit. Les pics du spectre pré-traité sont alors « comparés » à l'aide d'outil de classification à des données d'une base de connaissances construite à partir d'un ensemble de spectres de référence, chacun associé à un microorganisme identifié.

Plus particulièrement, l'identification de microorganismes par classification consiste classiquement :

■ en une première étape de détermination, à l'aide d'un apprentissage supervisé, d'un modèle de classification en fonction de spectres dits « d'apprentissage » de micro-organismes dont on connaît au préalable les espèces, le modèle de classification définissant un ensemble de règles distinguant ces différentes espèces parmi les spectres d'apprentissage.;
■ en une seconde étape d'identification d'un microorganisme particulier inconnu en :

o faisant l'acquisition d'un spectre de celui-ci ; et
o en appliquant au spectre acquis un modèle de prédiction construit à partir du modèle de classification afin de déterminer au moins une espèce auquel le microorganisme inconnu appartient.

Typiquement, un appareil d'identification par spectrométrie comporte un spectromètre et une unité de traitement d'information recevant les spectres mesurés et mettant en oeuvre la seconde étape précitée. La première étape est quant à elle mise en oeuvre par le constructeur de l'appareil qui détermine le modèle de classification et le modèle de prédiction et l'intègre dans la machine avant son exploitation par un client.

Les algorithmes du type de type machine à vecteur de support, ou algorithme « SVM » (pour « *support vector machine* ») sont des outils classiques d'apprentissage supervisé, particulièrement adaptés à l'apprentissage de modèles de classification de haute dimension visant à classer un nombre important d'espèces.

Toutefois, quand bien même les SVM seraient particulièrement adaptés à la haute dimension, l'établissement d'un modèle de classification par ces algorithmes est très complexe.

Tout d'abord, les algorithmes SMV classiquement utilisés font partis des algorithmes dits « à plat » qui considèrent les espèces à classer de manière équivalente et corolairement considèrent les erreurs de classification également comme équivalentes. Ainsi du point de vue algorithmique une erreur de classification entre deux bactéries proches à la même valeur qu'une erreur de classification entre une bactérie et un champignon. Il appartient alors à l'utilisateur, sur la base de ses connaissances des microorganismes utilisés pour produire les spectres d'apprentissage, sur la structure des spectres eux-mêmes, et sur la base de ses connaissances algorithmiques, de modifier l'algorithme SVM « à plat » utilisé pour minimiser la sévérité des erreurs de classification de celui-ci. Outre la difficulté que présente la modification d'un algorithme complexe, une telle modification est très dépendante de l'utilisateur lui-même.

Ensuite, quand bien même il existerait une dizaine ou plusieurs dizaines de spectres d'apprentissage différents pour chaque espèce de microorganismes pour construire le modèle de classification, ce nombre reste malgré tout très faible. Non seulement la variété représentée par les spectres d'apprentissage peut s'avérer très faible au regard de la variété totale de l'espèce, mais en outre un nombre limité d'instances a pour effet de mécaniquement exacerber la particularité de chaque spectre. De fait, le modèle de classification obtenu peut s'avérer imprécis pour certaines espèces et mettre en difficulté l'étape de prédiction ultérieure d'un microorganisme inconnu. Lors encore, il appartient à l'utilisateur d'interpréter les résultats rendus par l'identification pour connaître son degré de pertinence, et donc in fine en déduire un résultat exploitable.

**EXPOSE DE L'INVENTION**

Le but de la présente invention est de proposer un procédé d'identification de microorganismes par spectrométrie ou spectroscopie à base de modèle de classification obtenu par une méthode d'apprentissage supervisée de type SVM qui minimise la sévérité des erreurs d'identification, permettant ainsi une identification sensiblement plus fiable de microorganismes inconnus.

A cet effet l'invention a pour objet un Procédé d'identification par spectrométrie de microorganismes inconnus parmi un ensemble d'espèces de référence, comportant :

■ une première étape d'apprentissage supervisé d'un modèle de classification des espèces de référence, comportant :

o pour chaque espèce, l'acquisition d'un ensemble de spectres d'apprentissage de microorganismes identifiés appartenant à ladite espèce ;
o la transformation de chaque spectre d'apprentissage acquis en un ensemble de données d'apprentissage selon un format prédéterminé pour leur utilisation par un algorithme de type machine à vecteur de support multi-classe ; et
o la détermination du modèle de classification des espèces de référence en fonction des ensembles de données d'apprentissage au moyen dudit algorithme de type machine à vecteur de support multi-classe,

■ une seconde étape de prédiction d'un microorganisme inconnu à identifier comportant :

o l'acquisition d'un spectre du microorganisme inconnu ; et
o l'application d'un modèle de prédiction en fonction dudit spectre et du modèle de classification afin d'inférer au moins un type de micro-organisme auquel le microorganisme inconnu appartient.

Selon l'invention :

■ la transformation de chaque spectre d'apprentissage acquis comporte :

o la transformation du spectre en un vecteur de données représentatif d'une structure du spectre d'apprentissage ;
o la production de l'ensemble de données selon le format prédéterminé en réalisant le produit tensoriel du vecteur de données par un vecteur prédéterminé représentant de manière biunivoque la position de l'espèce de référence du microorganisme dans une représentation hiérarchique sous forme d'arbre des espèces de référence en termes d'évolution et/ou de phénotype clinique ;

■ et le modèle de classification est un modèle de classification de classes correspondant à des noeuds de l'arbre de la représentation hiérarchique, l'algorithme de type machine à vecteur de support multi-classe consistant à déterminer des paramètres du modèle de classification en résolvant un unique problème d'optimisation d'un critère exprimé en fonction des paramètres du modèle de classification sous des contraintes de marges comprenant des fonctions dites « de perte » quantifiant une proximité entre les noeuds de l'arbre.

En d'autres termes, l'invention introduit de manière spécifique une information *a priori* qui jusqu'alors n'a pas été considérée dans les algorithmes d'apprentissage supervisés utilisés dans la construction de modèles de classification pour l'identification de micro-organisme, à savoir une représentation hiérarchique sous forme d'arbre des espèces de microorganismes en termes d'évolution et/ou de phénotype clinique. Une telle représentation hiérarchique est par exemple un arbre taxinomique dont la structure est essentiellement guidée par l'évolution des espèces, et par conséquent qui contient de manière intrinsèque une notion de similarité ou de proximité entre espèces.

L'algorithme SVM n'est ainsi plus un algorithme « à plat », les espèces n'étant plus interchangeables. Corolairement, les erreurs de classification ne sont donc plus jugées identiques par l'algorithme. En établissement un lien entre les espèces à classer, le procédé selon l'invention prend ainsi en compte de manière explicite et/ou implicite le fait qu'elles possèdent des informations en commun, donc également des informations non communes, ce qui aide par conséquent à distinguer les espèces, et donc à minimiser les erreurs de classification ainsi que l'impact du nombre réduit de spectres d'apprentissage par espèce.

L'introduction de cette information *a priori* dans l'algorithme est réalisée au moyen d'une structuration des données et des variables grâce au produit tensoriel. Ainsi, la structure des données et des variables de l'algorithme associées à deux espèces est d'autant plus similaire que ses espèces sont proches en termes d'évolution et/ou de phénotype clinique. Comme les algorithmes SVM sont des algorithmes visant à optimiser une fonction de coût sous contraintes, l'optimisation tient donc nécessairement compte des similarités et des différences entre les structures associées aux espèces.

D'une certaine manière, il peut être argumenté que la proximité entre espèce est prise en compte de manière « qualitative » par la structuration des données et variables. Selon l'invention, la proximité entre espèce est également prise en compte de manière « quantitative » par un choix particulier des fonctions de perte qui interviennent dans la définition des contraintes de l'algorithme SVM. Cette proximité « quantitative » des espèces est par exemple déterminée en fonction d'une « distance » définie sur l'arbre des espèces de référence ou peut être déterminée de manière complètement indépendante de ce dernier, par exemple en fonction de besoin spécifique de l'utilisateur. Il en résulte donc une minimisation des erreurs de classification ainsi qu'un gain en robustesse de l'identification par rapport au manque de richesse des spectres d'apprentissage.

Enfin, le modèle de classification se rapporte à présent à la classification des noeuds de l'arbre de la représentation hiérarchique, racine et feuilles comprises, et non plus uniquement aux espèces. Notamment si au cours d'une prédiction mise en oeuvre sur le spectre d'un microorganisme inconnu, il est difficile de déterminer avec un degré de certitude minimal à quelle espèce appartient le microorganisme, la prédiction est capable d'identifier à quel groupe plus large (genre, famille, ordre, ...) de microorganismes appartient le microorganisme inconnu. Cette information précieuse peut par exemple être utilisée pour mettre en oeuvre d'autres types d'identifications microbiennes spécifiques audit groupe identifié.

Selon un mode de réalisation, des fonctions de perte associées à des couples de noeuds sont égales à des distances séparant les noeuds dans l'arbre de la représentation hiérarchique. De cette manière, l'algorithme est optimisé vis-à-vis dudit arbre, et les fonctions de perte ne dépendent pas du savoir faire et des connaissances de l'utilisateur.

Selon un mode de réalisation, des fonctions de perte associées à des couples de noeuds sont supérieures respectivement à des distances séparant les noeuds dans l'arbre de la représentation hiérarchique. Ainsi, un autre type d'information *a priori* peut être introduit dans la construction du modèle de classification. Notamment, la séparabilité algorithmique des espèces peut être forcée en choisissant des fonctions de perte dont la valeur est supérieure à la distance dans l'arbre.

Selon un mode de réalisation, les fonctions de perte sont calculées :

■ en réglant les fonctions de pertes à des valeurs initiales ;
■ en mettant en oeuvre au moins une itération d'un processus consistant à :

  o exécuter un algorithme de type machine à vecteur de support multi-classe de manière à calculer un modèle de classification en fonction de valeurs courantes des fonctions de perte ;
  o appliquer un modèle de prédiction en fonction du modèle de classification calculé et d'un ensemble de spectres de calibration de microorganismes identifiés appartenant aux espèces de référence, différent de l'ensemble de spectres d'apprentissage ;
  o calculer un critère de performance de la classification pour chaque espèces en fonction de résultats renvoyés par ladite application du modèle de prédiction à l'ensemble de spectres de calibration ; et
  o calculer de nouvelles valeurs courantes des fonctions de pertes en modifiant les valeurs courantes des fonctions de perte en fonction des critères de performance calculés.

Les fonctions de perte permettent notamment de régler la séparabilité des espèces vis-à-vis des spectres d'apprentissage et/ou de l'algorithme SVM utilisé. Il est notamment possible de détecter les espèces peu séparables et de mettre en oeuvre un algorithme qui modifie les fonctions de perte pour augmenter cette séparabilité.

Dans une première variante :

■ le calcul du critère de performance consiste en le calcul d'une matrice de confusion en fonction des résultats renvoyés par ladite application du modèle de prédiction ;
■ et les nouvelles valeurs courantes des fonctions de perte sont calculées en fonction de la matrice de confusion.

De cette manière, l'impact de l'introduction des informations taxinomiques et/ou de phénotype clinique contenues dans l'arbre de la représentation hiérarchique est évalué et les erreurs ou les défauts de classification restant sont minimisés en choisissant des fonctions de perte en fonction de ceux-ci.

Selon une seconde variante :

■ le calcul du critère de performance consiste en le calcul d'une matrice de confusion en fonction des résultats renvoyés par ladite application du modèle de prédiction ;
■ et les nouvelles valeurs courantes des fonctions de perte correspondent respectivement aux composantes d'une combinaison d'une première matrice de perte répertoriant des distances séparant les espèces de référence dans l'arbre de la représentation hiérarchique et d'une seconde matrice calculée en fonction de la matrice de confusion.

Tout comme la première variante, les erreurs et les défauts de classification restants sont corrigés tout en gardant dans les fonctions de pertes des informations quantitatives de distances entre espèces dans l'arbre.

Notamment, les valeurs courantes des fonctions de perte sont calculées selon la relation :

$$\Delta(y_i, k) = \alpha \times \Omega(y_i, k) + (1 - \alpha) \times \Delta_{confusion}(y_i, k)$$

où $\Delta(y_i, k)$ sont lesdites valeurs courantes des fonctions de perte pour les couples de noeuds $(y_i, k)$ de l'arbre, $\Omega(y_i, k)$ et $\Delta_{confustion}(y_i, k)$ sont respectivement les première et seconde matrices, et $\alpha$ est un scalaire compris entre 0 et 1. Plus particulièrement, le scalaire $\alpha$ est compris entre 0, 25 et 0, 75, notamment entre 0, 25 et 0, 5.

Une telle combinaison convexe permet à la fois une précision élevée de l'identification et une minimisation de la sévérité des erreurs d'identification.

Plus particulièrement, les valeurs initiales des fonctions de perte sont réglées à zéro pour des couples de noeuds différents et égal à 1 sinon.

Selon un mode de réalisation, une distance $\Omega$ séparant deux noeuds $n_1$, $n_2$ dans l'arbre de la représentation hiérarchique est déterminée selon la relation :

$$\Omega(n_1, n_2) = depth(n_1) + depth(n_2) - 2 \times depth(LCA(n_1, n_2))$$

où $depth(n_1)$ et $depth(n_2)$ sont respectivement la profondeur des noeuds $n_1$, $n_2$, et $depth(LCA(n_1, n_2))$ est la profondeur de l'ancêtre commun le plus proche $LCA(n_1, n_2)$ des noeuds $n_1$, $n_2$ dans ledit arbre. La distance $\Omega$ ainsi définie est la distance minimale définissable sur un arbre.

Selon un mode de réalisation, le modèle de prédiction est un modèle de prédiction des noeuds de l'arbre auquel appartient le micro-organisme inconnu à identifier. Ainsi, il est possible de prédire des noeuds ancêtres des feuilles correspondant aux espèces.

Selon un mode de réalisation, le problème d'optimisation est formulé selon les relations :

$$\min_{W, \xi_i} \frac{1}{2} \|W\|^2 + C \sum_{i=1}^{N} \xi_i$$

sous les contraintes :

$$\xi_i \geq 0, \forall i \in [1, N]$$

$$\langle W, \Psi(x_i, y_i) \rangle \geq \langle W, \Psi(x_i, k) \rangle + f(\Delta(y_i, k), \xi_i), \forall i \in [1, N], \forall k \in Y \setminus y_i$$

expressions dans lesquelles :

■ $N$ est le nombre de spectres d'apprentissage ;
■ $K$ est le nombre d'espèces de référence ;
■ $T$ est le nombre de noeuds dans l'arbre de la représentation hiérarchique et $Y = [1, T]$ est un ensemble d'entier référençant les noeuds de l'arbre de la représentation hiérarchique ;

■ $W \in \Re^{p \times T}$ est la concaténation $(w_1 w_2 ... w_T)^T$ de vecteurs de poids $w_1$, $w_2$, ..., $W \in \Re^p$ associés respectivement aux noeuds dudit arbre, $p$ étant la cardinalité des vecteurs représentatifs de la structure des spectres d'apprentissage ;
■ $C$ est un scalaire de réglage prédéterminé ;

- $\forall i \in [1, N], \xi_i$ est un scalaire ;

- $X = \{x_i\}$, $i \in [1,N]$ est un ensemble de vecteurs $x_i \in \mathfrak{R}^p$ représentatifs des spectres d'apprentissage ;
- $\forall i \in [1, N]$, $y_i$ est la référence du noeud dans l'arbre de la représentation hiérarchique correspondant à l'espèce de référence du vecteur d'apprentissage $x_i$ ;
- $\Psi(x, k) = x \otimes \Lambda(k)$, où :

  o $x \in \mathfrak{R}^p$ est un vecteur représentatif d'un spectre d'apprentissage;

  o $\Lambda(k) \in \mathfrak{R}^T$ est un vecteur prédéterminé représentant de manière biunivoque la position du noeud de référence $k \in Y$ dans l'arbre de la représentation hiérarchique ; et

  o $\otimes : \mathfrak{R}^p \times \mathfrak{R}^T \to \mathfrak{R}^{p \times T}$ est le produit tensoriel entre l'espace $\mathfrak{R}^p$ et l'espace $\mathfrak{R}^T$ ;

- $\langle W, \psi \rangle$ est le produit scalaire sur l'espace $\mathfrak{R}^{p \times T}$ ;
- $\Delta(y_i, k)$ est la fonction de perte associée au couple de noeuds de référence respective $y_i$ et $k$ dans l'arbre de la représentation hiérarchique ;
- $f(\Delta(y_i, k), \xi_i)$ est une fonction prédéterminée du scalaire $\xi_i$ et de la fonction de perte $\Delta(y_i, k)$ ; et
- le symbole « \ » désigne le symbole de l'exclusion.

Dans une première variante, la fonction $f(\Delta(y_i, k), \xi_i)$ est définie selon la relation $f(\Delta(y_i, k), \xi_i) = \Delta(y_i, k) - \xi_i$.

Dans une seconde variante, la fonction $f(\Delta(y_i, k), \xi_i)$ est définie selon la relation $f\big(\Delta(y_i, k), \xi_i\big) = 1 - \dfrac{\xi_i}{\Delta(y_i, k)}$ .

Notamment, l'étape de prédiction comporte :

- la transformation du spectre du microorganisme inconnu à identifier en un vecteur $x_m$ selon le format prédéterminé de l'algorithme de type machine à vecteur de support multi-classe ;
- l'application d'un modèle de prédiction selon les relations :

$$T_{ident} = \arg\max_k (s(x_m, k)) \quad k \in [1, T]$$

où $T_{ident}$ est la référence du noeud de la représentation hiérarchique identifié pour le microorganisme inconnu, $s(x_m, k) = \langle W, \Psi(x_m, k) \rangle$ et $\Psi(x_m, k) = x_m \otimes \Lambda(k)$ .

L'invention a également pour objet un dispositif d'identification d'un microorganisme par spectrométrie de masse, comprenant :

- un spectromètre apte à produire des spectres de masse de microorganismes à identifier ;
- une unité de calcul apte à identifier les microorganismes associés aux spectres produits par le spectromètre en mettant en oeuvre une étape de prédiction du type précité.

## BREVE DESCRIPTION DES FIGURES

L'invention sera mieux comprise à la lecture de la description qui suit, donnée uniquement à titre d'exemple, et réalisée en relation avec les dessins annexés, dans lesquels des références identiques désignent des éléments identiques ou analogues, et dans lesquels :

- la figure 1 est un organigramme d'un procédé d'identification selon l'invention ;
- la figure 2 est un exemple d'arbre de taxinomie hybride mélangeant par exemple des informations phénotypiques et évolutives;

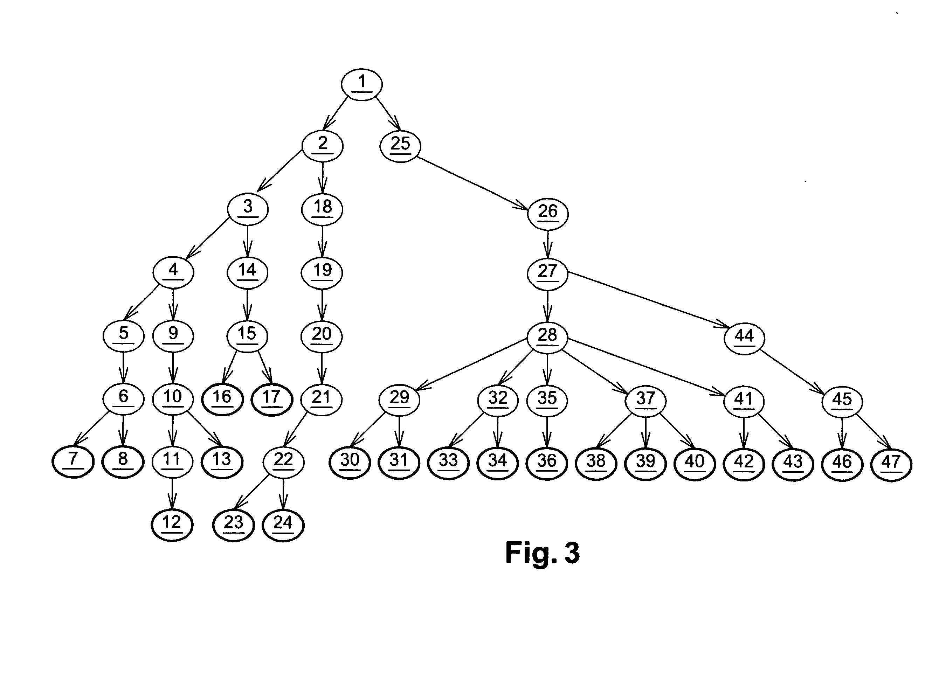

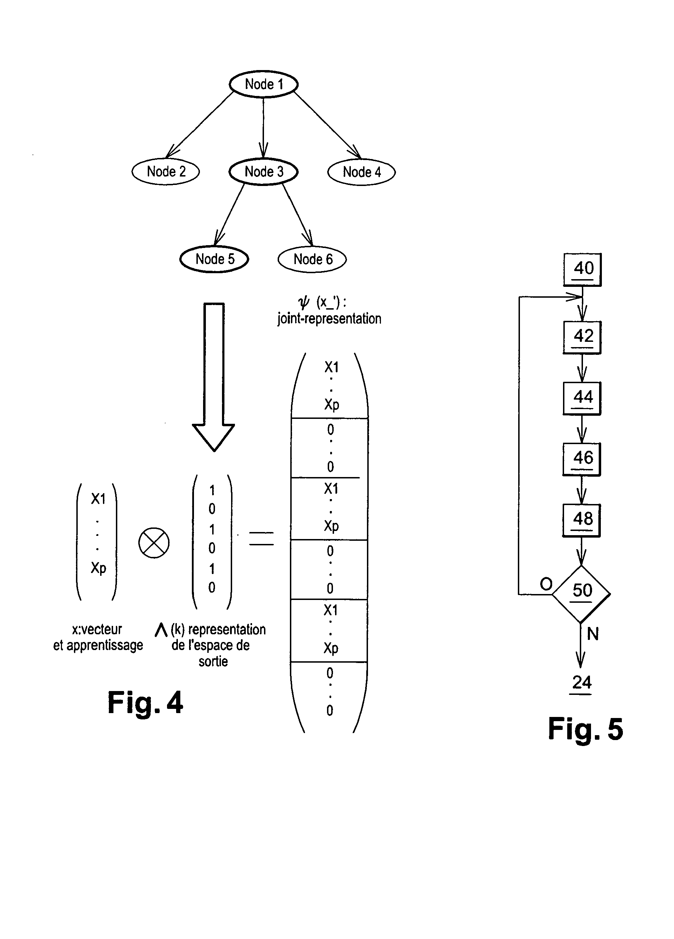

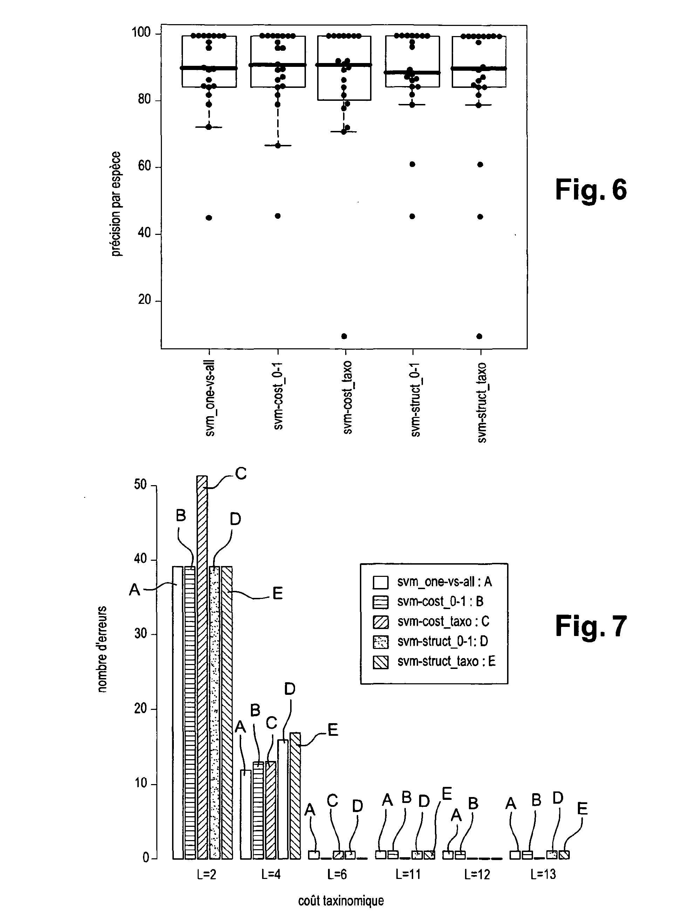

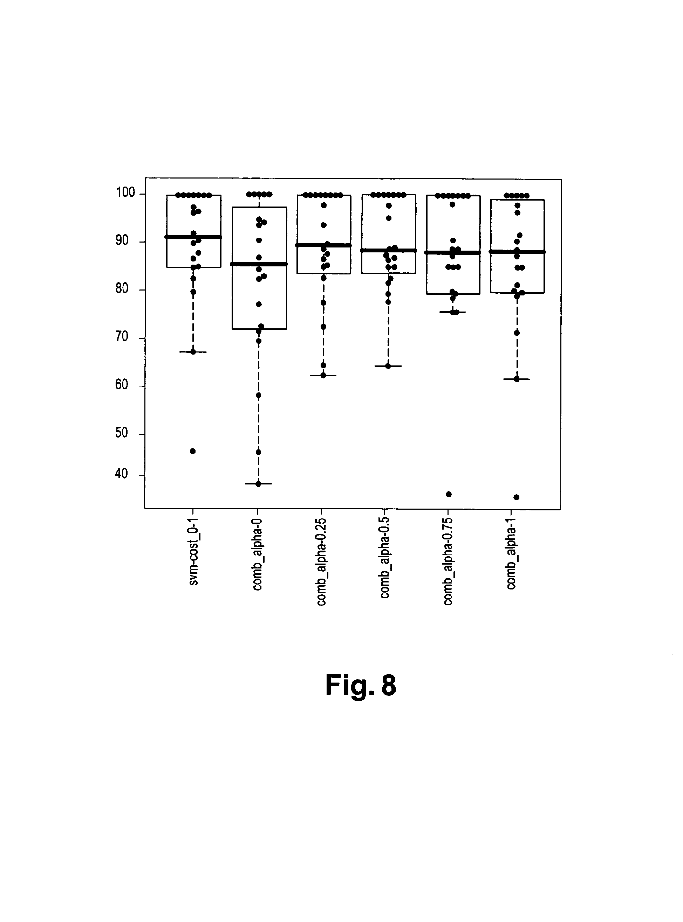

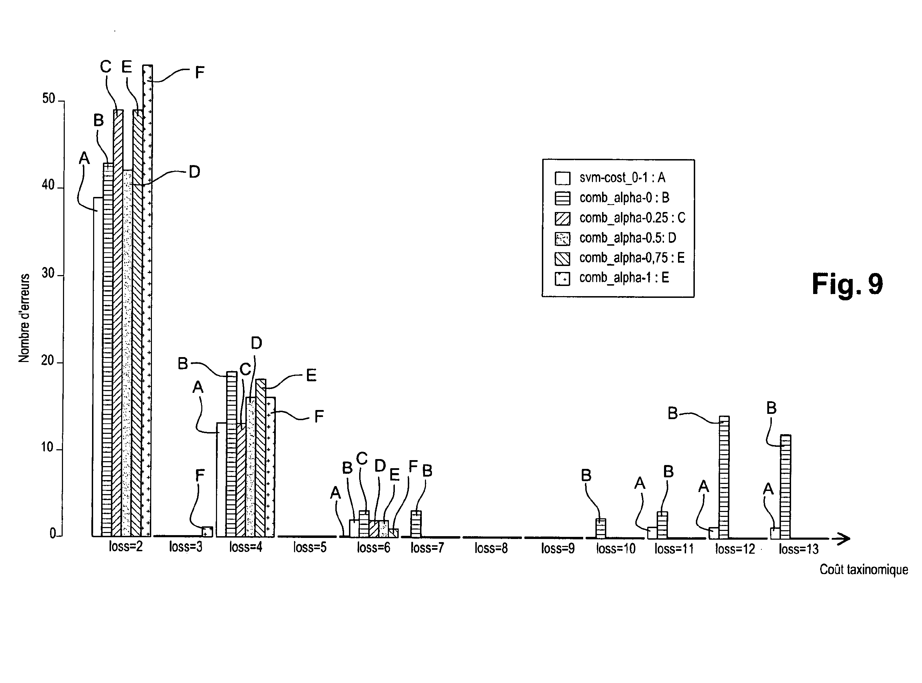

■ la figure 3 est un exemple d'arbre d'une représentation hiérarchique utilisé selon l'invention ;
■ la figure 4 est un exemple de génération d'un vecteur correspondant à la position d'un noeud dans un arbre ;
■ la figure 5 est un organigramme d'un procédé de calcul de fonctions de perte selon l'invention ;
■ la figure 6 est un tracé illustrant des précisions par espèce de différents algorithmes d'identification ;
■ la figure 7 est un tracé illustrant des coûts taxinomiques de erreurs de prédiction de ces différents algorithmes ;
■ la figure 8 est un tracé illustrant des précisions par espèce d'un algorithme utilisant des fonctions de perte égales à différentes combinaisons convexes d'une distance sur l'arbre de la représentation hiérarchique et d'une fonction de perte de confusion ; et
■ la figure 9 est un tracé des coûts taxinomiques des erreurs de prédictions pour les différentes combinaisons convexes.

## DESCRIPTION DETAILLEE DE L'INVENTION

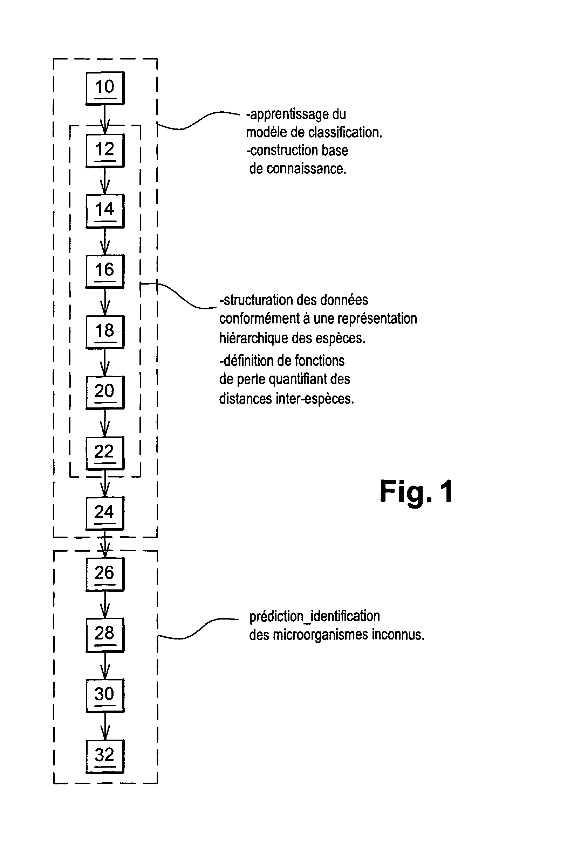

Il va à présent être décrit en relation avec l'organigramme de la figure 1, un procédé selon l'invention appliqué la spectrométrie MALDI-TOF.

Le procédé débute par une étape **10** d'acquisition d'un ensemble de spectres de masse d'apprentissage d'une nouvelle espèce de microorganisme à intégrer dans une base de connaissance, par exemple au moyen d'une spectrométrie de masse de type MALDI-TOF (acronyme de « _Matrix-assisted laser desorption/ionization time of flight_ »). La spectrométrie de masse MALDI-TOF est bien connue en soi et ne sera donc pas décrite plus en détail par la suite. On pourra, par exemple, se référer au document de Jackson O. Lay, « Maldi-tof spectrometry of bacteria », Mass Spectrometry Reviews, 2001, 20, 172-194. Les spectres acquis sont ensuite prétraités, afin notamment de débruiter ceux-ci et ôter la leur ligne de base, d'une manière connue en soi.

Une identification des pics présents dans les spectres acquis est alors réalisée en 12, par exemple au moyen d'un algorithme de détection de pics basé sur la détection de maxima locaux. Une liste des pics pour chaque spectre acquis, comportant la localisation et l'intensité des pics du spectre, est ainsi produite.

De manière avantageuse, les pics sont identifiés dans la gamme de Thomson $[m_{min} ; m_{max}]$ prédéterminée, de préférence la gamme $[m_{min} ; m_{max}] = [3000; 17000]$ Thomson. En effet, il a été observé que les informations suffisantes à l'identification des micro- organismes sont regroupées dans cette gamme de rapport masse sur charge, et qu'il n'est donc pas besoin de tenir compte d'une gamme plus large.

Le procédé se poursuit, en **14,** par une étape de quantification, ou « _binning_ ». Pour ce faire la gamme $[m_{min} ; m_{max}]$ est subdivisée en intervalles de largueurs prédéterminées, par exemple constante, et pour chaque intervalle comprenant plusieurs pics, un seul pic est conservé, avantageusement le pic présentant la plus forte intensité. Un vecteur est ainsi produit pour chaque spectre mesuré. Chaque composante du vecteur correspond à un intervalle de la quantification et a pour valeur l'intensité du pic conservé pour cet intervalle, la valeur « 0 » signifiant qu'aucun pic n'a été détecté dans cet intervalle.

En variante, les vecteurs sont « binarisés » en posant la valeur d'une composante du vecteur à « 1 » lorsqu'un pic est présent dans l'intervalle correspondant, et à « 0 » lorsqu'aucun pic n'est présent dans cet intervalle. Ceci a pour effet de rendre plus robuste la calibration des algorithmes de classification réalisée ultérieurement. Les inventeurs ont en effet noté que l'information pertinente notamment pour l'identification d'une bactérie est contenue essentiellement dans l'absence et/ou la présence de pics, et que l'information d'intensité est moins pertinente. En outre, on observe que l'intensité est une grandeur très variable d'un spectre à l'autre et/ou d'un spectromètre à un autre. Du fait de cette variabilité, il est difficile de prendre en compte les valeurs brutes d'intensité dans les outils de classification.

Parallèlement, les vecteurs de pics de spectres d'apprentissage, ci- après « vecteurs d'apprentissage », sont mémorisés dans la base de connaissance. La base de connaissance répertorie ainsi $K$ espèces de microorganismes, dites « espèces de référence », et un ensemble $X = \{x_i\}_{i \in [1,N]}$ de $N$ spectres d'apprentissage $x_i \in \mathfrak{R}^p$, $i \in [1, N]$, où $p$ est le nombre de pics retenus pour les spectres de masse.

Parallèlement, ou consécutivement, les $K$ espèces répertoriées sont classées, en **16**, selon une représentation hiérarchique sous forme d'arbre des espèces de référence en termes d'évolution et/ou de phénotype clinique.

Dans une première variante, la représentation hiérarchique est une représentation taxinomique du vivant appliquée aux espèces de référence répertoriées. Comme cela est connu en soi, la taxinomie du vivant est une classification hiérarchique du vivant qui classe chaque organisme vivant selon l'ordre suivant, du moins spécifique au plus spécifique : domaine, règne, phylum, classe, ordre, famille, genre espèce. La taxinomie utilisée est par exemple celle établie par le « _National Center for Biotechnology Information_ » (NCBI). La taxinomie du vivant comprend ainsi de manière implicite des données d'évolution, des microorganismes proches au niveau évolution comportant plus de composants en commun que des microorganismes plus éloignés du point de vue évolution. Il ressort de ceci que la « proximité » en termes d'évolution a un impact sur la « proximité » des spectres.

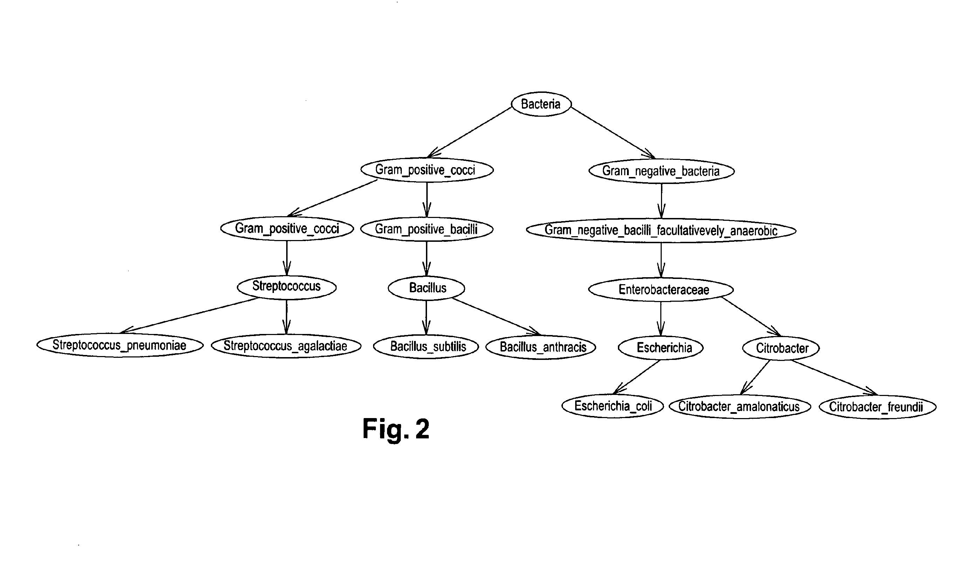

Dans une seconde variante, la représentation hiérarchique est une représentation taxinomique « hybride » obtenue en prenant en compte des caractéristiques phylogénique, par exemple des caractéristiques d'évolution des espèces, et des caractéristiques phénotypiques, comme par exemple le GRAM +/- des bactéries qui se base sur l'épaisseur /perméabilité de leur membranes, leur caractéristique aérobie ou anaérobie. Une telle représentation est par exemple illustrée à la figure 2 pour les bactéries.

D'une manière générale, l'arbre de la représentation hiérarchique est une représentation graphique reliant des noeuds terminaux, ou « feuilles », correspondants aux espèces à un noeud « racine » par un unique chemin constitué de noeuds intermédiaires.

Dans une étape 18 suivante, les noeuds de l'arbre, ou « taxons », sont numérotés par la par des entiers $k \in Y = [1, T]$, où T est le nombre de noeuds dans l'arbre, feuilles et racine comprises, et l'arbre est transformé en un ensemble $\Lambda = \{\Lambda(k)\}_{k \in [1, T]}$ de vecteurs binaires $\Lambda(k) \in \Re^T$.

Plus particulièrement, les $T$ noeuds de l'arbre sont numérotés respectivement de 1 à $T$, par exemple conformément aux différents chemins depuis la racine vers les feuilles, comme cela est illustré sur l'arbre de la figure 3 qui répertorie 47 noeuds dont 20 espèces. Les composantes des vecteurs $\Lambda(k)$ correspondent alors aux noeuds ainsi numérotés, la première composante des vecteurs $\Lambda(k)$ correspondant au noeud numéroté « 1 », la deuxième composante correspondant au noeud numéroté « 2 » et ainsi de suite. Les composantes d'un vecteur $\Lambda(k)$ correspondant aux noeuds dans le chemin depuis le noeud $k$ jusqu'à la racine de l'arbre, noeud $k$ et racine comprise, sont posées égales à un, et les autres composantes du vecteur $\Lambda(k)$ sont posées égales à zéro. La figure 4 illustre la génération des vecteurs $\Lambda(k)$ pour un arbre simplifié de 5 noeuds. Le vecteur $\Lambda(k)$ représente ainsi de manière biunivoque, ou unique, la position du noeud $k$ dans l'arbre de la représentation hiérarchique, et la structure du vecteur $\Lambda(k)$ représente les liens d'ascendance du noeud $k$. Dit autrement, l'ensemble $\Lambda = \{\Lambda(k)\}_{k \in [1, T]}$ est une représentation vectorielle de l'ensemble des chemins entre la racine et les noeuds de l'arbre de la représentation hiérarchique.

D'autres représentations vectorielles de l'arbre conservant ces liens sont évidemment possibles.

Pour bien comprendre ce qui suit, il est introduit les notations suivantes. Chaque vecteur d'apprentissage $x_i$ correspond à une espèce de référence particulière labélisée par un entier $y_i \in [1, T]$, à savoir le numéro de la feuille correspondante dans l'arbre de la représentation hiérarchique. Par exemple, le $10^{ième}$ vecteur d'apprentissage $x_{10}$ correspond à l'espèce représenté par la feuille numérotée « 24 » de l'arbre de la figure 3, auquel cas $y_{10} = 24$. La notation $y_i$ se réfère ainsi au numéro, ou « label », de l'espèce du spectre dans l'ensemble [1, $T$], la cardinalité de l'ensemble $E = \{y_i\}$ des références $y_i$ étant bien entendu égal à au nombre K d'espèces de référence. Ainsi en se référant par exemple à la figure 3, $E = \{7, 8, 12, 13, 16, 17, 23, 24, 30, 31, 33, 34, 36, 38, 39, 40, 42, 43, 46, 47\}$. Lorsqu'un entier de $Y = [1, T]$, par exemple l'entier « $k$ », est utilisé directement dans les relations qui suivent, cet entier se réfère au noeud numéroté « $k$ » de l'arbre, indépendamment des vecteurs d'apprentissage $x_i$.

Dans une étape suivante **20,** de nouveaux vecteurs « d'apprentissage structurés » $\Psi(x_i, k) \in \Re^{p \times T}$ sont produits conformément aux relations :

$$\Psi(x_i, k) = x_i \otimes \Lambda(k) \quad \forall i \in [1, N], \forall k \in [1, T] \quad (1)$$

où $\otimes : \Re^p \times \Re^T \to \Re^{p \times T}$ est le produit tensoriel entre l'espace $\Re^p$ et l'espace $\Re^T$. Un vecteur $\Psi(x_i, k)$ est ainsi un vecteur qui comprend une concaténation de $T$ blocs de dimension $p$ dont les blocs correspondants aux composantes égales à l'unité du vecteur $\Lambda(k)$ sont égaux au vecteur $x_i$ et dont les autres blocs sont égaux au vecteur nul $0_p$ de $\Re^p$. En se référant à nouveau à l'exemple de la figure 4, le vecteur $\Lambda(5)$ correspondant au noeud numéroté « 5 » est égal à

$$\begin{pmatrix} 1 \\ 0 \\ 1 \\ 0 \\ 1 \\ 0 \end{pmatrix} \text{ et le vecteur } \Psi(x_i, 5) \text{ est égal à } \begin{pmatrix} x_i \\ 0_p \\ x_i \\ 0_p \\ x_i \\ 0_p \end{pmatrix}$$

On constate ainsi que plus des noeuds sont proches dans l'arbre de la représentation hiérarchique, plus leurs vecteurs structurés partagent des blocs non nuls communs. A contrario, plus des noeuds sont éloignées, moins leurs vecteurs structurés partagent de blocs non nuls en commun, ces constations s'appliquant donc en particulier aux feuilles représentant les espèces de référence.

Dans une étape suivante **22,** il est calculé des fonctions de perte d'un algorithme de type SVM multi-classe structuré appliqué à l'ensemble des noeuds de l'arbre de la représentation hiérarchique.

Plus particulièrement, un algorithme SVM multi-classes structuré conformément à la représentation hiérarchique selon l'invention est défini selon les relations :

$$\min_{W,\xi_i} \frac{1}{2}\|W\|^2 + C\sum_{i=1}^{N}\xi_i \qquad (2)$$

sous les contraintes :

$$\xi_i \geq 0, \forall i \in [1,N] \qquad (3)$$

$$\langle W, \Psi(x_i,y_i)\rangle \geq \langle W, \Psi(x_i,k)\rangle + f(\Delta(y_i,k),\xi_i), \forall i \in [1,N], \forall k \in Y \setminus y_i \qquad (4)$$

expressions dans lesquelles :

■ $W \in \Re^{p \times T}$ est la concaténation $(w_1 w_2 ... w_T)^T$ de vecteurs de poids $w_1, w_2, ..., w_T \in \Re^p$ associés respectivement aux noeuds $y_i$ de l'arbre ;
■ $C$ est un scalaire de réglage prédéterminé ;
■ $\forall i \in [1, N]$ , $\xi_i$ est un scalaire ;

■ $\langle W, \psi \rangle$ est le produit scalaire, ici sur l'espace $\Re^{p \times T}$ ;
■ $\Delta(y_i, k)$ est une fonction de perte de perte définie pour le couple formé de l'espèce ayant pour référence $y_i$ et du noeud ayant pour référence $k$ ;
■ $f(\Delta(y_i, k), \xi_i)$ est une fonction prédéterminée du scalaire $\xi_i$ et de la fonction de perte $\Delta(y_i, k)$ ; et
■ le symbole « \ » désignant le symbole de l'exclusion, l'expression « $\forall k \in Y \setminus y_i$ » signifiant ainsi « tous les noeuds de l'ensemble $Y$ sauf le noeud de référence $y_i$ ».

Comme on peut le constater, la proximité entre espèces, telle que codée par la représentation hiérarchique, et telle qu'introduite dans la structure des vecteurs d'apprentissage structurés, est prise en compte via les contraintes. Notamment plus les espèces sont proches dans l'arbre, et plus leurs données sont couplées. Les espèces de référence ne sont donc plus considérées comme interchangeables par l'algorithme selon l'invention, contrairement aux algorithmes SVM multi-classes classiques, qui ne considèrent aucune hiérarchisation entre les espèces et considèrent celles-ci comme étant interchangeables.

En outre, l'algorithme SVM multi-classes structuré selon l'invention prend en compte de manière quantitative la proximité entre les espèces de référence au moyen des fonctions de perte $\Delta(y_i,k)$.

Selon une première variante, la fonction f est définie selon la relation :

$$f(\Delta(y_i,k),\xi_i) = \Delta(y_i,k) - \xi_i \qquad (5)$$

Selon une seconde variante, la fonction f est définie selon la relation :

$$f(\Delta(y_i,k),\xi_i) = 1 - \frac{\xi_j}{\Delta(y_i,k)} \qquad (6)$$

Dans un mode de réalisation avantageux, les fonctions de perte $\Delta$ ($y_{i,}k$) sont égales à une distance $\Omega$ ($y_i$, $k$) définie sur l'arbre de la représentation hiérarchique selon relation :

$$\Delta(y_i,k) = \Omega(y_i,k) = depth(y_i) + depth(k) - 2 \times depth(LCA(y_i,k)) \quad (7)$$

où $depth$ ($y_i$) et $depth$ ($k$) sont respectivement la profondeur des noeuds $y_i$, et $k$ dans ledit arbre, et $depth$ ($LCA$ ($y_i$, $k$) ) est la profondeur du noeud ascendant, ou noeud « ancêtre », commun le plus proche $LCA$ ($y_i$, $k$) des noeuds $y_i$ et $k$ dans ledit arbre. La profondeur d'un noeud est par exemple définie comme étant le nombre de noeuds qui le sépare du noeud racine.

En variante, les fonctions de perte $\Delta(y_i,k)$ sont de nature différente de celle de la représentation hiérarchique. Ces fonctions sont par exemple définies par l'utilisateur en fonction d'une autre représentation hiérarchique, de son savoir faire et/ou de résultats algorithmiques, comme cela sera expliqué plus en détail par la suite.

Une fois les fonctions de pertes calculées, le procédé selon l'invention se poursuit par la mise en oeuvre, en **24**, de l'algorithme SVM multi-classes, tel que défini aux relations (2), (3), (4), (5) ou (2), (3), (4), (6).

Le résultat produit par l'algorithme est ainsi le vecteur $W$ qui est le modèle de classification des noeuds de l'arbre, déduit de la combinaison des informations contenues dans les vecteurs d'apprentissage $x_i$, du positionnement de leur espèces de références associées dans l'arbre, de l'information de proximité entre espèces contenues dans la représentation hiérarchique, et de l'information de distance entre espèces contenue dans les fonctions de pertes. Plus particulièrement, chaque vecteur de poids $w_l$, $l \in$ [1, $T$], représente le vecteur normal d'un hyperplan de $\Re^p$ représentant une frontière entre les instances du noeud « $l$ » de l'arbre et les instances des autres noeuds $k \in$ [1, $T$] \/ de l'arbre.

Les étapes **12** à **24** d'apprentissage du modèle de classification sont mises en oeuvre une fois dans un premier système informatique. Le modèle de classification $W = (w_1 w_2 ... w_T)^T$, ainsi que les vecteurs $\Lambda$ ($k$), sont ensuite mémorisés dans un système d'identification de microorganismes comprenant un spectromètre de masse de type MALDI- TOF et une unité de traitement informatique connectée au spectromètre. L'unité de traitement reçoit les spectres de masse acquis par le spectromètre et met en oeuvre des règles de prédiction déterminant, sur la base du modèle $W$ et des vecteurs $\Lambda$ ($k$), à quels noeuds de l'arbre de la représentation hiérarchique sont associés les spectres de masse acquis par le spectromètre de masse.

En variante, la prédiction est réalisée sur un serveur distant accessible par un utilisateur, par exemple au moyen d'un ordinateur personnel connecté au réseau internet auquel est également connecté le serveur. L'utilisateur charge des spectres de masse non traités obtenus par un spectromètre de masse de type MALDI-TOF sur le serveur, ce dernier met alors en oeuvre l'algorithme de prédiction et renvoie les résultats de l'algorithme à l'ordinateur de l'utilisateur.

Plus particulièrement, pour l'identification d'un microorganisme inconnu, le procédé comporte une étape **26** d'acquisition d'un ou plusieurs spectres de masse de celui-ci, une étape **28** de prétraitement des spectres acquis ainsi qu'une étape **30** de détection des pics des spectres et de détermination d'un vecteur de pics $x_m \in \Re^p$, tel que par exemple décrites précédemment en relation avec les étapes **10** à **14.**

Dans une étape **32** suivante, un vecteur structuré est calculé pour chaque noeud de l'arbre de la représentation hiérarchique $k \in Y$= [1, $T$] selon la relation :

$$\Psi(x_m,k) = x_m \otimes \Lambda(k) \quad (8)$$

puis un score associé au noeud $k$ est calculé selon la relation :

$$s(x_m,k) = \langle W, \Psi(x_m,k) \rangle \quad (9)$$

Le noeud de l'arbre $T_{ident} \in$ [1, $T$] identifié du microorganisme inconnu est alors par exemple celui qui correspond au score le plus élevé :

$$T_{ident} = \arg\max_k (s(x_m,k)) \quad k \in [1, T] \quad (10)$$

D'autres modèles de prédiction sont bien entendu possibles.

Outre le score associé au taxon identifié $T_{ident}$, les scores des noeuds ancêtres et des noeuds descendants si existants, du taxon $T_{ident}$ sont également calculés par l'algorithme de prédiction. Ainsi par exemple, si le score du taxon $T_{ident}$ est jugé faible par l'utilisateur, ce dernier dispose de scores associés aux noeuds ancêtres et donc d'informations supplémentaires plus fiables.

Il vient d'être décrit un mode de réalisation particulier de l'invention dans lequel les fonctions de pertes $\Delta(y_i, k)$ sont calculées en fonction d'une distance minimale définie dans l'arbre de la représentation hiérarchique.

Il va à présent être décrit d'autres variantes de calcul des fonctions de pertes $\Delta(y_i, k)$.

Dans une première variante, les fonctions de pertes définies à la relation (7) sont modifiées en fonction d'informations *a priori* permettant d'obtenir un modèle de classification plus robuste et/ou de faciliter la résolution du problème d'optimisation défini par les relations (2), (3) et (4). Par exemple, la fonction de perte $\Delta(y_i, k)$ d'un couple de noeuds ($y_i$, $k$) peut être choisie faible, notamment inférieure à la distance $\Omega(y_i, k)$, signifiant qu'il est toléré des erreurs d'identification entre ces deux noeuds. Relâcher des contraintes sur un ou plusieurs couple(s) d'espèces revient mécaniquement à accroitre les contraintes sur les autres couples d'espèces, l'algorithme étant donc réglé pour différentier plus fortement les autres couples. De manière analogue, la fonction de perte $\Delta(y_i, k)$ d'un couple de noeuds ($y_i, k$) peut être choisie très élevée, notamment supérieure à la distance $\Omega(y_i, k)$, pour forcer l'algorithme à différentier les noeuds ($y_i, k$), et donc minimiser les erreurs d'identification entre celles-ci. Notamment, il est possible relâcher ou renforcer des contraintes pourtant sur des couples d'espèces de référence au moyen de leurs fonctions de pertes respective.

Dans une seconde variante, illustrée à l'organigramme de la figure 5, le calcul des fonctions de perte $\Delta(y_i, k)$ est réalisé de manière automatique en fonction de performances estimées de l'algorithme SVM mis en oeuvre pour calculer le modèle de classification $W$.

Le procédé de calcul des fonctions de pertes $\Delta(y_i, k)$ débute par le choix, en **40,** de valeurs initiales pour celles-ci. Par exemple, $\Delta(y_i, k) = 0$ lorsque $y_i = k$, et $\Delta(y_i, k) = 1$ lorsque $y_i \neq k$, les fonctions f se réduisant ainsi à $f(\Delta(y_i, k), \xi_i) = 1 - \xi_i$. D'autres valeurs initiales sont bien évidemment possibles pour les fonctions de perte, les fonctions $f(\xi_i) = 1 - \xi_i$ apparaissant dans les contraintes des algorithmes exposés ci-dessous étant alors remplacée par les fonctions $f(\Delta(y_i, k), \xi_i)$ de la relation (5) ou (6) avec les valeurs initiales des fonctions de perte.

Le procédé de calcul se poursuit par l'estimation des performances de l'algorithme SVM pour les fonctions de perte $\Delta(y_i, k)$ choisies. Cette estimation comporte :

■ l'exécution, en **42,** d'un algorithme SVM multi-classes en fonction des valeurs des fonctions de perte de manière à calculer un modèle de classification ;

■ l'application, en **44,** d'un modèle de prédiction sur la base du modèle de classification calculé, le modèle de prédiction étant appliqué à un ensemble $\{\tilde{x}_i\}$ de vecteurs de calibration $x_i \in \mathfrak{R}^p$ de la base de connaissance. Les vecteurs de calibration $\tilde{x}_i$ sont produits de manière similaire aux vecteurs d'apprentissage $x_i$ à partir de spectres associés aux espèces de référence, chaque vecteur $\tilde{x}_i$ étant associé à la référence $\tilde{y}_i$ de l'espèce de référence correspondante; et

■ la détermination, en **46,** d'une matrice de confusion en fonction des résultats de la prédiction.

Les vecteurs de calibration $\tilde{x}_i$ sont par exemple acquis simultanément aux vecteurs de d'apprentissage $x_i$. Notamment, pour chaque espèce de référence, les spectres associés à celle-ci sont répartis en un ensemble d'apprentissage et un ensemble de calibration desquels sont produits respectivement les vecteurs d'apprentissage et les vecteurs de calibration.

Le procédé de calcul des fonctions de perte se poursuit, en **48,** par la modification des valeurs des fonctions de perte en fonction de la matrice de confusion calculée. Les fonctions de perte obtenues sont alors utilisées par l'algorithme SVM de calcul du modèle de classification final $W$, ou bien un test est mis en oeuvre en **50** pour savoir si de nouvelles valeurs des fonctions de perte sont calculées en mettant en oeuvre les étapes **42, 44, 46, 48** en fonction valeurs modifiées des fonctions de perte lors de l'étape **48.**

Dans un premier exemple du procédé de calcul des fonctions de perte, l'étape **42** correspond à l'exécution d'un algorithme SVM est un algorithme du type « un contre tous », communément désigné sous l'expression « *one-versus-all* ». Cet algorithme est non hiérarchisé et considère uniquement les espèces de référence, celles-ci étant référencées par des entiers $k \in [1, K]$, et résout un problème d'optimisation pour chacune des espèces de référence $k$ selon les relations :

$$\min_{w_k, \xi_i} \frac{1}{2} \left\| w_k \right\|^2 + C \sum_{i=1}^{N} \xi_i \quad (11)$$

sous les contraintes :

$$\xi_i \geq 0, \forall i \in [1, N] \qquad (12)$$

$$q_i \left( \langle w_k, x_i \rangle + b_k \right) \geq 1 - \xi_i \qquad \forall i \in [1, N] \quad (13)$$

expressions dans lesquelles :

- $w_k \in \Re^p$ est un vecteur de poids et $b_k \in \Re$ est un scalaire ; et
- $q_i \in \{-1, 1\}$ avec $q_i = 1$ si $i = k$, et $q_i = -1$ si $i \neq k$ .

Le modèle de prédiction est donné selon la relation suivante et appliqué, lors de l'étape **44,** à chacun des vecteurs de calibration $\tilde{x}_i$ :

$$G(\tilde{x}_i) = \arg\max_k \langle w_k, \tilde{x}_i \rangle + b_k \quad k \in [1, K] \qquad (14)$$

Une matrice de confusion inter-espèce $C_{espèce} \in \Re^K \times \Re^K$ est alors calculée, lors de l'étape **46,** selon la relation :

$$C_{espèce}(i,k) = FP(i,k) \, \forall i, k \in [1, K] \qquad (15)$$

où $FP(i,k)$ est le nombre de vecteurs de calibration de l'espèce $i$ prédit par le modèle de prédiction comme appartenant à l'espèce $k$.

Toujours en **46,** une matrice de confusion inter-espèce normalisée $\tilde{C}_{espèce} \in \Re^K \times \Re^K$ est ensuite calculée selon la relation :

$$\tilde{C}_{espèce}(i,k) = \frac{C_{espèce}(i,k)}{N_i} \times 100 \quad (16)$$

où $N_i$ est le nombre de vecteurs de calibration pour l'espèce de référence $i$ .

Enfin l'étape **46** se termine par le calcul d'une matrice de confusion inter- noeud normalisée $C_{taxo} \in \Re^T \times \Re^T$ en fonction de la matrice de confusion normalisée $\tilde{C}_{espèce}$. Par exemple, un schéma de propagation des valeurs $\tilde{C}_{espèce}(i, k)$ depuis les feuilles vers la racine est utilisé pour calculer les valeurs $\tilde{C}_{taxo}(i, k)$ des couples $(i, k)$ de noeuds différents des espèces de référence. Notamment, pour un couple de noeuds $(i, k) \in [1, T]^2$ de l'arbre de la représentation hiérarchique pour lequel il est déjà calculé une composante de la matrice $\tilde{C}_{taxo}(i^C, k^C)$ pour chaque couple de noeuds $(i^C, k^C)$ de l'ensemble $\{i^C\} \times \{k^C\}$, où $\{i^C\}$ et $\{k^C\}$ sont respectivement les ensembles des noeuds « enfants » des noeuds $i$ et $k,$ la composante de la matrice $\tilde{C}_{taxo}(i^C, k^C)$ pour le couple $(i, k)$ est posée égale à la moyenne des composantes $\tilde{C}_{taxo}(i^C, k^C)$ .

Lors de l'étape **48,** la fonction de perte $\Delta(y_i, k)$ de chaque couple de noeuds $(y_i, k)$ est calculée en fonction de la matrice de confusion inter-noeud normalisée $\tilde{C}_{taxo}$.

Selon une première option de l'étape **48,** la fonction de perte $\Delta(y_i, k)$ est calculée selon la relation :

$$\Delta(y_i,k)=\begin{cases} 0 & si\ y_i = k \\ 1+\lambda\times\tilde{C}_{taxo}(y_i,k) & si\ y_i \neq k \end{cases} \qquad (17)$$

où $\lambda\geq 0$ est un scalaire prédéterminé commandant la contribution de la matrice de confusion $\tilde{C}_{taxo}$ dans la fonction de perte.

Selon une deuxième option de l'étape **48,** la fonction de perte $\Delta(y_i,k)$ est calculée selon la relation :

$$\Delta(y_i,k)=\begin{cases} 0 & si\ y_i = k \\ 1+\beta\times\left\lceil\dfrac{\tilde{C}_{taxo}(y_i,k)}{l}\right\rceil & si\ y_i \neq k \end{cases} \qquad (18)$$

où $\lceil\bullet\rceil$ est l'arrondi à l'entier supérieur, $\beta\geq 0$ et $l>0$ sont des scalaires prédéterminés réglant la contribution de la matrice de confusion $\tilde{C}_{taxo}$ dans la fonction de perte. Par exemple en posant $l$=10, la matrice de confusion $\tilde{C}_{taxo}$ contribue à hauteur de $\beta$ pour chaque tranche de 10% de confusion entre les noeuds $(y_i,k)$.

Selon une troisième option de l'étape **48,** une première composante $\Delta_{confusion}(y_i, k)$ de la fonction de perte $\Delta(y_i, k)$ est calculée selon la relation (17) ou (18), puis la fonction de perte $\Delta(y_i,k)$ est calculée selon la relation :

$$\Delta(y_i,k)=\alpha\times\Omega(y_i,k)+(1-\alpha)\times\Delta_{confusion}(y_i,k) \qquad (19)$$

où $0\leq\alpha\leq 1$ est un scalaire réglant un compromis entre une fonction de perte uniquement déterminée au moyen d'une matrice de confusion et une fonction de perte uniquement déterminée au moyen d'une distance dans l'arbre de la représentation hiérarchique.

Dans un deuxième exemple du procédé de calcul des fonctions de perte, l'étape **42** correspond à l'exécution d'un algorithme SVM multi- classes qui résout un unique problème d'optimisation pour toutes les espèces de référence $k\in[1, K]$, chaque vecteur d'apprentissage $x_i$ étant associé à son espèce de référence numérotée par un entier $y_i\in[1, K]$, selon les relations

$$\min_{w_k,\xi_i}\frac{1}{2}\sum_{k=1}^{K}\|w_k\|^2+C\sum_{i=1}^{N}\xi_i \qquad (20)$$

sous les contraintes :

$$\xi_i\geq 0,\ \forall i\in[1,N] \qquad (21)$$

$$\langle w_{y_i},x_i\rangle\geq\langle w_k,x_i\rangle+1-\xi_i \quad \forall i\in[1,N],\forall k\in[1,K]\setminus y_i \qquad (22)$$

où $\forall k\in[1, K]$, $w_k\in\mathfrak{R}^p$ vecteur de poids associé à l'espèce $k$.

Le modèle de prédiction est donné selon la relation suivante et appliqué, lors de l'étape **44,** à chacun des vecteurs de calibration $\tilde{x}_i$ :

$$G(\tilde{x}_i)=\arg\max_{k}\langle w_k,\tilde{x}_i\rangle \quad k\in[1,K] \qquad (23)$$

Les étapes **46** et **48** du deuxième exemple sont identiques aux étapes **46** et **48** du premier exemple.

Dans un troisième exemple du procédé de calcul des fonctions de perte, l'étape **42** correspond à l'exécution de SVM multi-classes structuré à base de représentation hiérarchique selon l'invention relations (2), (3), (4), (5) ou (2), (3), (4), (6). Lors de l'étape **44,** le modèle de prédiction selon la relation suivante est alors appliqué à chacun des vecteurs

de calibration $\tilde{x}_i$ :

$$G(\tilde{x}_i) = \arg\max_k \langle W, \Psi(\tilde{x}_i, k) \rangle \quad k \in E \quad (29)$$

où $E = \left\{ y_k^{espèce} \right\}$ est l'ensemble des références des noeuds de l'arbre de la représentation hiérarchique correspondant aux espèces de référence.

Une matrice de confusion inter-espèce $C_{espèce} \in \mathfrak{R}^K \times \mathfrak{R}^K$ est alors déduite des résultats de la prédiction sur les vecteurs de calibration $\tilde{x}_i$ et le procédé de calcul des fonctions de perte se poursuit de manière identique à celle du premier exemple.

Bien entendu, la matrice de confusion peut être calculée en fonction de résultats de prédictions portants sur tous les taxons de l'arbre.

Il a été décrit des modes de réalisation dans lesquels l'algorithme SVM mis en oeuvre pour calculer le modèle de classification est un modèle SVM multi-classes structuré à base de représentation hiérarchique, notamment un algorithme selon les relations (2), (3), (4), (5) ou selon les relations (2), (3), (4), (6).

Le principe des fonctions de perte $\Delta(y_i, k)$ qui quantifient une proximité *a priori* entre des classes envisagées par l'algorithme, à savoir des noeuds de l'arbre de la représentation hiérarchique dans les modes de réalisation décrits précédemment, s'appliquent également à des algorithmes SVM multi-classes ne se basant pas sur une représentation hiérarchique. Pour de tels algorithmes, les classes considérées sont les espèces de référence représentées dans les algorithmes par des entiers $k \in [1, K]$, et les fonctions de perte sont définies uniquement pour les couples d'espèces de référence, et donc pour les couples $(y_i, k) \in [1, K]^2$.

Notamment, dans un autre mode de réalisation, l'algorithme SVM utilisé pour calculer le modèle de classification est l'algorithme SVM multi-classes selon les relations (20), (21) et (22) en remplaçant la fonction $f(\xi_i) = 1 - \xi_i$ de la relation (22) par la fonction $f(\Delta(y_i, k), \xi_i)$ selon la relation (5) ou la relation (6), à savoir selon les relations (20), (21) et (22bis) :

$$\langle w_{y_i}, x_i \rangle \geq \langle w_k, x_i \rangle + f(\Delta(y_i, k), \xi_i) \quad \forall i \in [1, N], \forall k \in [1, K] \setminus y_i \quad (22\text{bis})$$

Le modèle de prédiction appliqué pour identifier l'espèce d'un micro-organisme inconnu est alors le modèle selon la relation (23).

Il va à présent être décrit des résultats expérimentaux du procédé selon l'invention, selon les conditions expérimentales suivantes :

■ 571 spectres de bactéries obtenus par un spectromètre de masse de type MALDI-TOF;
■ les bactéries appartiennent à 20 espèces de référence différentes et représentent plus de 200 souches différentes ; et
■ les 20 espèces sont hiérarchiquement organisées en un arbre taxinomique de 47 noeuds tel qu'illustré à la figure 3 ;
■ les vecteurs d'apprentissage et de calibration sont produits en fonction des spectres de mass et répertorient chacun l'intensité de 1300 pics en fonction du ratio masse-sur-charge. On a donc $x_i \in \mathfrak{R}^{1300}$.

Les performances du procédé selon l'invention sont évaluées à l'aide d'une validation croisée définie de la manière suivante :

■ pour chaque souche, un ensemble de vecteurs d'apprentissage est défini en retirant de l'ensemble total des vecteurs d'apprentissage les vecteurs correspondant à la souche ;
■ pour chaque ensemble ainsi obtenu, il est calculé un modèle de classification sur la base d'un algorithme de type SVM tel que décrit précédemment; et
■ un modèle de prédiction associé au modèle de classification obtenu est appliqué aux vecteurs correspondant à la souche retirée de l'ensemble des vecteurs d'apprentissage.

Par ailleurs, différents indicateurs sont pris en compte pour évaluer les performances du procédé :

■ la micro précision qui est le taux de spectres correctement classés ;

■ des précisions par espèce, une précision pour une espèce étant le taux de spectres correctement classés pour cette espèce ;

■ la macro précision qui est la moyenne des précisions par espèce. Contrairement à la micro précision, la macro précision est moins sensible à la cardinalité des ensembles de vecteurs d'apprentissage respectivement associés aux espèces de référence;

■ le coût « taxinomique » d'une prédiction, qui est la longueur du chemin le plus court dans l'arbre de la représentation hiérarchique entre l'espèce de référence d'un spectre et l'espèce prédite pour ce spectre, par exemple définie comme étant égale à la distance $\Omega(y_i, k)$ selon la relation (7). Contrairement à la micro précision, les précisions par espèce et la macro précision, qui considèrent les erreurs de prédiction comme étant d'importance égale, le coût taxinomique permet de quantifier la sévérité de chaque erreur de prédiction.

Les algorithmes suivants ont été analysés et comparés:

■ « SVM_one-vs-all » : algorithme selon les relations (11), (12), (13), (14) :
■ « SVM_cost_0-1 » : algorithme selon les relations (20), (21), (22), (23) ;
■ « SVM_ cost_ taxo » : algorithme selon les relations (20), (21), (22bis), et (23) avec $f(\Delta(y_i, k), \xi_i)$ définie selon les relations (6) et (7) ;
■ « SVM_ struct_ 0- 1 » : algorithme selon les relations (2), (3), (4), (8)- (10) avec $f(\Delta(y_i, k), \xi_i) = 1 - \xi_i$ ;
■ « SVM_ struct_ taxo » : algorithme selon les relations (2), (3), (4), (8)- (10) avec $f(\Delta(y_i, k), \xi_i)$ définie selon les relations (6) et (7) .

Le paramètre C retenu pour chaque de ces algorithmes est celui permettant les meilleures micro précision et macro précision.

Le tableau ci-dessous répertorie pour chacun de ces algorithmes la micro précision et la macro précision. La figure 6 illustre la précision par espèce de chacun des algorithmes, la figure 7 illustre le nombre d'erreur de prédiction en fonction du coût taxinomique de celles-ci de chacun des algorithmes.

| Algorithme SVM | Micro précision | Macro précision |
|---|---|---|
| SVM_one-vs-all | 90,4 | 89,2 |
| SVM_cost_0-1 | 90,4 | 89,0 |
| SVM_cost_taxo | 88,6 | 86,0 |
| SVM_struct_0-1 | 89,2 | 88,5 |
| SVM_struct_taxo | 90,4 | 89,2 |

A la vue de ces résultats, et notamment du tableau ci-dessous et de la figure 6, on remarque qu'à la fois la représentation des données conformément à la représentation hiérarchique et les fonctions de perte ont une incidence sur la précision des prédictions, tant en terme de micro précision que de macro précision. On note à cet égard que l'algorithme « SVM_struct_taxo » de l'invention fait au moins jeux égal que l'algorithme classique du type « one-versus-all ». Toutefois, au regard de la figure 7, on remarque les erreurs de prédiction des algorithmes sont de sévérités différentes. Notamment, les algorithmes « SVM_one-vs-all » et « SVM_cost_0-1 », qui ne prennent en compte aucune représentation hiérarchique entre les espèces de référence, produisent des erreurs de prédiction de sévérité importante. L'algorithme réalisant le moins d'erreurs sévères est l'algorithme « SVM_cost_taxo », aucune erreur de coût taxinomique supérieur à 4 ayant été détectée. Par contre, l'algorithme « SVM_cost_taxo » est moins performant en termes de micro précision et de macro-précision.

A la vue de ce qui précède, on en déduit donc que l'introduction d'informations *a priori* sous la forme d'une représentation hiérarchique, notamment taxinomique et/ou phénotypique clinique, des espèces de référence et de distances quantitatives entre les espèces sous la forme de fonctions de perte permet de gérer le compromis entre d'une part la précision globale de l'identification de microorganismes inconnus et d'autre part la sévérité des erreurs d'identification.

Des analyses ont également été menées sur des fonctions de perte égale à une combinaison convexe de la distance sur l'arbre et fonction de perte de confusion selon la relation (19), plus particulièrement, pour l'algorithme « SVM_ cost_ taxo_ conf» selon les relations (20), (21), (22bis) . La fonction $f(\Delta(y_i, k), \xi_i)$ est définie selon la relation (6) et les fonctions de perte $\Delta(y_i, k)$ sont calculées en mettant en oeuvre le deuxième exemple du procédé de calcul des fonctions de perte $\Delta(y_i, k)$, avec $\Delta(y_i, k)$ définie selon les relations (18) et (19) en remplaçant la matrice de confusion inter- noeuds par la matrice de confusion inter- espèce. L'algorithme « SVM_ cost_ taxo_ conf » a été mise en oeuvre pour différentes valeurs du paramètre $\alpha$, à savoir les valeurs 0, 0, 25, 0, 5, 0, 75 et 1, le paramètre $\beta$ dans la relation (18) étant égal à 1, et le paramètre C dans la relation (20) étant égal à 1000. Le résultat de cette analyse sont illustrés aux figures 8 et 9, qui illustrent respectivement les précisions par espèce et les coûts taxinomiques pour les différentes valeurs du paramètre $\alpha$. Sur ces figures sont également illustrées, à des fins de comparaison, les précisions par espèce et les coûts taxinomique de l'algorithme « SVM_ cost_ 0/1 »

Comme on peut le noter sur ces figures, lorsque le paramètre $\alpha$ s'approche de un, les fonctions de perte étant donc sensiblement définies uniquement par la distance sur l'arbre de la représentation hiérarchique, la précision diminue et la sévérité des erreurs augmente. De même, lorsque le paramètre $\alpha$ s'approche de zéro, les fonctions de perte étant sensiblement définies uniquement à partir d'une matrice de confusion, la précision par espèce diminue et la sévérité des erreurs augmente.

Par contre, pour des valeurs du paramètre $\alpha$ comprises dans la gamme [0, 25; 0, 75], et notamment dans la gamme [0, 25; 0, 5], on observe une précision plus importante, la précision par espèce la plus faible étant supérieure de 60% à la précision par espèce la plus faible de l'algorithme SVM_ cost_ 0/1. On observe également une diminution sensible des erreurs sévères de prédiction, notamment de coût taxinomique supérieur à 6. En outre, on observe que pour des valeurs de $\alpha$ proches de 0, 5, notamment pour la valeur 0, 5 illustrée aux figures, le nombre d'erreurs de coût taxinomique égal à 2 est réduit par rapport au nombre d'erreurs de même coût avec des valeurs de $\alpha$ proche de 0, 25.

Des analyses préliminaires montrent un impact similaire pour un algorithme « SVM_ struct_ taxo_ conf » mettant en oeuvre les relations (2), (3), (4), (8)- (10) avec comme fonction $f(\Delta(y_i, k), \xi_i)$ celle définie à la relation (6) et comme fonctions de perte $\Delta(y_i, k)$ celles calculées en mettant en oeuvre le deuxième exemple du procédé de calcul des fonctions de perte $\Delta(y_i, k)$ en utilisant les relations (18) et (19).

Il a été décrit des modes de réalisation appliqués à la spectrométrie de masse de type MALDI-TOF. Ces modes de réalisation s'appliquent à tout type de spectrométrie et spectroscopie, notamment la spectrométrie vibrationnelle, et la spectroscopie par auto-fluorescence, seule la génération des vecteurs d'apprentissage, notamment le prétraitement des spectres, pouvant varier.

De même, il a été décrit des modes de réalisation dans lesquelles les spectres utilisés pour produire les données d'apprentissage ne présentent aucune structure.

Or, les spectres sont par nature « structurés », c'est- à- dire que leurs composantes, les pics, ne sont par interchangeables. Notamment, un spectre comprend un ordonnancement intrinsèque, par exemple en fonction du ratio masse- sur- charge pour la spectrométrie de masse ou en fonction de la longueur d'onde pour la spectrométrie vibrationnelle, et une molécule ou un composé organique peut donner naissance à plusieurs pics.

Selon la présente invention, la structure intrinsèque des spectres est également prise en compte en mettant en oeuvre des algorithmes de type SVM non linéaires utilisant des fonctions noyaux $K(x,y)$ symétriques et définies positives quantifiant la similarité de structure d'une couple de spectres $(x,y)$. Les produits scalaires entre deux vecteurs apparaissant dans les algorithmes SVM décrits ci-dessus sont alors remplacés par lesdites fonctions noyaux $K(x,y)$. Pour plus de détails, on pourra par exemple se référer au chapitre 11 du document « Kernel Methods for Pattern Analysis » de John Shawe-Taylor & Nello Cristianini - Cambridge University Press, 2004.

## Revendications

**1.** Procédé d'identification par spectrométrie de microorganismes inconnus parmi un ensemble d'espèces de référence, comportant :

■ une première étape d'apprentissage supervisé d'un modèle de classification des espèces de référence, comportant :

    o pour chaque espèce, l'acquisition d'un ensemble de spectres d'apprentissage de microorganismes identifiés appartenant à ladite espèce ;
    o la transformation de chaque spectre d'apprentissage acquis en un ensemble de données d'apprentissage selon un format prédéterminé pour leur utilisation par un algorithme de type machine à vecteur de support multi-classe ; et
    o la détermination du modèle de classification des espèces de référence en fonction des ensembles de données d'apprentissage au moyen dudit algorithme de type machine à vecteur de support multi-classe,

■ une seconde étape de prédiction d'un microorganisme inconnu à identifier comportant :

    o l'acquisition d'un spectre du microorganisme inconnu ; et

o l'application d'un modèle de prédiction en fonction dudit spectre et du modèle de classification afin d'inférer au moins un type de micro-organisme auquel le microorganisme inconnu appartient, *caractérisé*:

■ en ce que la transformation de chaque spectre d'apprentissage acquis comporte :

o la transformation du spectre en un vecteur de données représentatif d'une structure du spectre d'apprentissage ;
o la production de l'ensemble de données selon le format prédéterminé en réalisant le produit tensoriel du vecteur de données par un vecteur prédéterminé représentant de manière biunivoque la position de l'espèce de référence du microorganisme dans une représentation hiérarchique sous forme d'arbre des espèces de référence en termes d'évolution et/ou de phénotype clinique ;

■ et en ce que le modèle de classification est un modèle de classification de classes correspondant à des noeuds de l'arbre de la représentation hiérarchique, l'algorithme de type machine à vecteur de support multi-classe consistant à déterminer des paramètres du modèle de classification en résolvant un unique problème d'optimisation d'un critère exprimé en fonction des paramètres du modèle de classification sous des contraintes de marges comprenant des fonctions dites « de perte » quantifiant une proximité entre les noeuds de l'arbre.

2. Procédé d'identification selon la revendication 1, *caractérisé* **en ce que** des fonctions de perte associées à des couples de noeuds sont égales à des distances séparant les noeuds dans l'arbre de la représentation hiérarchique.

3. Procédé d'identification selon la revendication 1 ou 2, *caractérisé* **en ce que** des fonctions de perte associées à des couples de noeuds sont supérieures respectivement à des distances séparant les noeuds dans l'arbre de la représentation hiérarchique.

4. Procédé d'identification la revendication 1, 2 ou 3, *caractérisé* **en ce que** les fonctions de perte sont calculées :

■ en réglant les fonctions de pertes à des valeurs initiales ;
■ en mettant en oeuvre au moins une itération d'un processus consistant à :

o exécuter un algorithme de type machine à vecteur de support multi-classe de manière à calculer un modèle de classification en fonction de valeurs courantes des fonctions de perte ;
o appliquer un modèle de prédiction en fonction du modèle de classification calculé et d'un ensemble de spectres de calibration de microorganismes identifiés appartenant aux espèces de référence, différent de l'ensemble de spectres d'apprentissage ;
o calculer un critère de performance de la classification pour chaque espèces en fonction de résultats renvoyés par ladite application du modèle de prédiction à l'ensemble de spectres de calibration ; et
o calculer de nouvelles valeurs courantes des fonctions de pertes en modifiant les valeurs courantes des fonctions de perte en fonction des critères de performance calculés.

5. Procédé d'identification selon la revendication 4, *caractérisé* **en ce que** :

■ en que le calcul du critère de performance consiste en le calcul d'une matrice de confusion en fonction des résultats renvoyés par ladite application du modèle de prédiction ;
■ et **en ce que** les nouvelles valeurs courantes des fonctions de perte sont calculées en fonction de la matrice de confusion.

6. Procédé d'identification selon la revendication 4, *caractérisé* **en ce que** :

■ en que le calcul du critère de performance consiste en le calcul d'une matrice de confusion en fonction des résultats renvoyés par ladite application du modèle de prédiction ;
■ et **en ce que** les nouvelles valeurs courantes des fonctions de perte correspondent respectivement aux composantes d'une combinaison d'une première matrice de perte répertoriant des distances séparant les espèces de référence dans l'arbre de la représentation hiérarchique et d'une seconde matrice calculée en fonction de la matrice de confusion.

7. Procédé d'identification selon la revendication 6, *caractérisé* **en ce que** les valeurs courantes des fonctions de perte sont calculées selon la relation :

$$\Delta(y_i, k) = \alpha \times \Omega(y_i, k) + (1 - \alpha) \times \Delta_{confusion}(y_i, k)$$

où $\Delta$ ($y_i, k$) sont lesdites valeurs courantes des fonctions de perte pour les couples de noeuds ($y_i$, $k$) de l'arbre, $\Omega$ ($y_i$, $k$) et $\Delta_{confustion}$ ($y_i$, $k$) sont respectivement les première et seconde matrices, et $\alpha$ est un scalaire compris entre 0 et 1.

8. Procédé d'identification selon la revendication 7, *caractérisé* **en ce que** le scalaire $\alpha$ est compris entre 0,25 et 0,75, notamment entre 0,25 et 0,5.

9. Procédé d'identification selon l'une des revendications 4 à 8, *caractérisé* **en ce que** les valeurs initiales des fonctions de perte sont réglées à zéro pour des couples de noeuds différents et égal à 1 sinon.

10. Procédé d'identification selon l'une quelconque des revendications précédentes, *caractérisé* **en ce qu'**une distance $\Omega$ séparant deux noeuds $n_1$, $n_2$ dans l'arbre de la représentation hiérarchique est déterminée selon la relation :

$$\Omega(n_1, n_2) = depth(n_1) + depth(n_2) - 2 \times depth(LCA(n_1, n_2))$$

où *depth* ($n_1$) et *depth* ($n_2$) sont respectivement la profondeur des noeuds $n_1$, $n_2$, et *depth* (*LCA* ($n_1$, $n_2$) ) est la profondeur de l'ancêtre commun le plus proche *LCA* ($n_1$, $n_2$) des noeuds $n_1$, $n_2$ dans ledit arbre.

11. Procédé d'identification selon l'une quelconque des revendications précédentes, *caractérisé* **en ce que** le modèle de prédiction est un modèle de prédiction des noeuds de l'arbre auquel appartient le micro-organisme inconnu à identifier.

12. Procédé d'identification selon l'une quelconque des revendications précédentes, *caractérisé* **en ce que** le problème d'optimisation est formulé selon les relations :

$$\min_{W, \xi_i} \frac{1}{2} \|W\|^2 + C \sum_{i=1}^{N} \xi_i$$

sous les contraintes :

$$\xi_i \geq 0, \forall i \in [1, N]$$

$$\langle W, \Psi(x_i, y_i) \rangle \geq \langle W, \Psi(x_i, k) \rangle + f(\Delta(y_i, k), \xi_i), \forall i \in [1, N], \forall k \in Y \setminus y_i$$

expressions dans lesquelles :

- ◼ $N$ est le nombre de spectres d'apprentissage ;
- ◼ $K$ est le nombre d'espèces de référence ;
- ◼ T est le nombre de noeuds dans l'arbre de la représentation hiérarchique et $Y=[1, T]$ est un ensemble d'entier référençant les noeuds de l'arbre de la représentation hiérarchique ;

- ◼ $W \in \Re^{p \times T}$ est la concaténation ($w_1 w_2 ... w_T$) $^T$ de vecteurs de poids $w_1$, $w_2$, ..., $W \in \Re^p$ associés respectivement aux noeuds dudit arbre, $p$ étant la cardinalité des vecteurs représentatifs de la structure des spectres d'apprentissage ;
- ◼ $C$ est un scalaire de réglage prédéterminé ;

■ $\forall i \in [1, N]$, $\xi_i$ est un scalaire ;

■ $X = \{x_i\}$, $i \in [1,N]$ est un ensemble de vecteurs $x_i \in \Re^p$ représentatifs des spectres d'apprentissage ;

   ■ $\forall i \in [1, N]$, $y_i$ est la référence du noeud dans l'arbre de la représentation hiérarchique correspondant à l'espèce de référence du vecteur d'apprentissage $x_i$ ;
   ■ $\Psi(x, k) = x \otimes \Lambda(k)$, où :

   o $x \in \Re^p$ est un vecteur représentatif d'un spectre d'apprentissage;

   o $\Lambda(k) \in \Re^T$ est un vecteur prédéterminé représentant de manière biunivoque la position du noeud de référence $k \in Y$ dans l'arbre de la représentation hiérarchique ; et

   o $\otimes : \Re^p \times \Re^T \to \Re^{p \times T}$ est le produit tensoriel entre l'espace $\Re^p$ et l'espace $\Re^T$;

■ $\langle W, \psi \rangle$ est le produit scalaire sur l'espace $\Re^{p \times T}$;
   ■ $\Delta(y_i, k)$ est la fonction de perte associée au couple de noeuds de référence respective $y_i$ et $k$ dans l'arbre de la représentation hiérarchique ;
   ■ $f(\Delta(y_i, k), \xi_i)$ est une fonction prédéterminée du scalaire $\xi_i$ et de la fonction de perte $\Delta(y_i, k)$ ; et
   ■ le symbole « \ » désigne le symbole de l'exclusion.

**13.** Procédé d'identification selon la revendication 12, *caractérisé* **en ce que** la fonction $f(\Delta(y_i, k), \xi_i)$ est définie selon la relation :

$$f\big(\Delta(y_i, k), \xi_i\big) = \Delta(y_i, k) - \xi_i$$

**14.** Procédé d'identification selon la revendication 12, *caractérisé* **en ce que** la fonction $f(\Delta(y_i, k), \xi_i)$ est définie selon la relation :

$$f\big(\Delta(y_i, k), \xi_i\big) = 1 - \frac{\xi_i}{\Delta(y_i, k)}$$

**15.** Procédé d'identification selon la revendication 12, 13, ou 14, *caractérisé* en ce l'étape de prédiction comporte :

   ■ la transformation du spectre du microorganisme inconnu à identifier en un vecteur $x_m$ selon le format prédéterminé de l'algorithme de type machine à vecteur de support multi-classe ;
   ■ l'application d'un modèle de prédiction selon les relations :

$$T_{ident} = \arg\max_k \big(s(x_m, k)\big) \quad k \in [1, T]$$

où $T_{ident}$ est la référence du noeud de la représentation hiérarchique identifié pour le microorganisme inconnu, $s(x_m, k) = \langle W, \Psi(x_m, k) \rangle$ et
$\Psi(x_m, k) = x_m \otimes \Lambda(k)$.

**16.** Dispositif d'identification d'un microorganisme par spectrométrie de masse, comprenant :

   ■ un spectromètre apte à produire des spectres de masse de microorganismes à identifier ;

■ une unité de calcul apte à identifier les microorganismes associés aux spectres produits par le spectromètre en mettant en oeuvre une étape de prédiction conforme à l'une quelconque des revendications précédentes.

-apprentissage du
modèle de classification.
-construction base
de connaissance.

-structuration des données
conformément à une représentation
hiérarchique des espèces.
-définition de fonctions
de perte quantifiant des
distances inter-espèces.

**Fig. 1**

prédiction_identification
des microorganismes inconnus.

**Fig. 2**

Fig. 3

$\psi$ (x_') : joint-representation

x:vecteur et apprentissage

$\bigwedge$ (k) representation de l'espace de sortie

**Fig. 4**

**Fig. 5**

Fig. 6

Fig. 7

Fig. 8

Fig. 9

**RAPPORT DE RECHERCHE EUROPEENNE**

Numéro de la demande

EP 12 30 5402

Europäisches Patentamt
European Patent Office
Office européen des brevets

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | T. VILLMANN ET AL: "Classification of mass-spectrometric data in clinical proteomics using learning vector quantization methods", BRIEFINGS IN BIOINFORMATICS, vol. 9, no. 2, 1 janvier 2007 (2007-01-01), pages 129-143, XP055054943, ISSN: 1467-5463, DOI: 10.1093/bib/bbn009 * page 130, * * page 131, colonne de gauche, ligne 19 - ligne 44 * * page 134, colonne de droite, ligne 41 - page 136, colonne de droite, ligne 10; figure 2 * | 1,2,16 | INV. G06K9/62 |
| Y | ----- ELENA N. ILINA ET AL: "Application of matrix-assisted laser desorption/ionization time-of-flight mass spectrometry for the study of Helicobacter pylori", RAPID COMMUNICATIONS IN MASS SPECTROMETRY, vol. 24, no. 3, 1 février 2010 (2010-02-01), pages 328-334, XP055054942, ISSN: 0951-4198, DOI: 10.1002/rcm.4394 * figure 4 * ----- | 1,2,16 | DOMAINES TECHNIQUES RECHERCHES (IPC) G06K |

Le présent rapport a été établi pour toutes les revendications

| Lieu de la recherche | Date d'achèvement de la recherche | Examinateur |
|---|---|---|
| La Haye | 28 février 2013 | Chateau, Jean-Pierre |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **JACKSON O. LAY.** Maldi-tof spectrometry of bacteria. *Mass Spectrometry Reviews,* 2001, vol. 20, 172-194

- **JOHN SHAWE-TAYLOR ; NELLO CRISTIANINI.** Kernel Methods for Pattern Analysis. Cambridge University Press, 2004